Europäisches Patentamt

European Patent Office

Office européen des brevets

(11) Numéro de publication: **0 217 689 B1**

(12) **FASCICULE DE BREVET EUROPEEN**

(45) Date de publication de fascicule du brevet: **22.04.92**  (51) Int. Cl.⁵: **A61B 8/06**

(21) Numéro de dépôt: **86401798.3**

(22) Date de dépôt: **11.08.86**

---

(54) **Sonde à ultrasons implantable.**

---

(30) Priorité: **12.08.85 FR 8512297**

(43) Date de publication de la demande:
**08.04.87 Bulletin 87/15**

(45) Mention de la délivrance du brevet:
**22.04.92 Bulletin 92/17**

(84) Etats contractants désignés:
**AT BE CH DE FR GB IT LI LU NL SE**

(56) Documents cités:
EP-A- 0 092 080    FR-A- 1 574 194
FR-A- 1 601 372    FR-A- 2 173 115
FR-A- 2 352 286    FR-A- 2 461 486
FR-A- 2 507 075    FR-A- 2 543 817
US-A- 4 541 433

WESCON TECHNICAL PAPERS, vol. 19, no.
20/4, 16-19 septembre 1975, pages 1-9, North
Hollywood, US; J.W. KNUTTI et al.: "Clinical
application of the pulsed Doppler ultrasonic
blood flowmeter"

(73) Titulaire: **VIRBAC S.A.**
**1ère Avenue 2 065 m, L.I.D.**
**F-06516 Carros Cedex(FR)**

(72) Inventeur: **Compos, André Charles**
**3, passage Privé**
**F-93500 Pantin(FR)**

(74) Mandataire: **Derambure, Christian**
**Cabinet Bouju Derambure (Bugnion) S.A. 55,**
**rue Boissonade**
**F-75014 Paris(FR)**

---

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention concerne une sonde à ultrasons implantable dans le corps humain ou animal.

L'invention est plus particulièrement adaptée à l'exploration ultrasonore d'un organe au contact duquel elle est appliquée, par exemple à la mesure par effet Doppler de la vélocité du sang à l'intérieur d'un vaisseau sanguin, tel que l'aorte. L'invention permet également de mesurer le diamètre du vaisseau sanguin, qui permet de calculer sa section. Par suite, on pourra ainsi calculer le débit du sang à l'intérieur du vaisseau. Cependant, l'invention pourra être utilisée dans d'autres domaines de l'industrie pour mesurer des vitesses et débits de fluides par effet Doppler, ou pour explorer d'autres organes qu'un vaisseau sanguin et autrement que par effet Doppler (par exemple, pour mesurer l'épaisseur de parois cardiaques ...).

On connaît déjà des sondes périvasculaires à ultrasons à effet Doppler. Ces sondes émettent une onde acoustique ultrasonore dans un fluide qui réfléchit cette onde. Le signal réfléchi reçu par la sonde a une fréquence légèrement différente de celle de l'onde émise, et cette différence est fonction de la vitesse du fluide. Ces sondes périvasculaires connues comportent des moyens piézo-électriques émetteurs/récepteurs connectés électriquement à des appareils externes fournissant l'énergie et le signal d'émission, analysant le signal réfléchi ... Ces derniers appareils externes ne font pas partie de l'invention.

Le brevet français 1 601 372 décrit un capteur du type piézo-électrique destiné aux mesures de vélocité sanguine en vue du calcul du débit sanguin, par sondage ultrasonore dans lequel une des faces de la plaquette en céramique piézo-électrique non tournée vers le milieu de propagation est au contact d'une masse de matière, notamment du caoutchouc, mousse ou résine chargée, assurant un découplage acoustique vis-à-vis du milieu de propagation. Un tel capteur peut être fixé à l'extrémité d'un cathéter, ou inséré dans un capot du type périvasculaire. Dans ce dernier cas, on prévoit une fixation au vaisseau grâce à une bande de dacron (marque déposée), et une masse de gel placée sur la face émettrice de la plaquette pour adapter l'impédance avec la paroi vasculaire. La sonde ainsi réalisée est essentiellement en altuglas (marque déposée), ou autre matériau classiquement transparent aux ultrasons. Une telle sonde connue n'offre pas toutes les qualités, notamment d'étanchéité, permettant son implantation dans un organisme vivant à long terme.

Le brevet français 1 574 194 décrit également une sonde périvasculaire à effet Doppler dans laquelle chaque transducteur est noyé au sein d'un support et recouvert sur sa face opposée à celle du support, destinée à être appliquée contre le milieu à sonder, d'une couche de matière à haute impédance acoustique. Cette sonde connue n'est cependant pas implantable dans un organisme vivant à long terme.

Le brevet français FR 2 352 286 concerne une sonde périvasculaire à effet Doppler dont les dispositifs qui permettent son fonctionnement sont intégralement implantables, et que l'on peut commander de l'extérieur de l'organisme par voie électromagnétique. Une telle sonde est difficilement utilisable en pratique. En effet, l'ensemble est trop volumineux pour pouvoir être implanté à long terme dans un organisme vivant, et nécessite une source d'énergie implantée dont on devra prévoir le remplacement périodiquement. De plus, son extraction nécessite une intervention chirurgicale complexe, délicate et coûteuse.

Dans un premier mode de réalisation des sondes périvasculaires connues, celles-ci comportent un élément piézo-électrique émetteur, et un élément piézo-électrique récepteur. Dans ce cas, l'émission d'ondes ultrasonores est continue. Dans un second mode de réalisation de ces sondes, celles-ci fonctionnent par effet Doppler pulsé. Dans ce cas, on émet les ondes ultrasonores de façon pulsée intermittente. On n'emploie alors qu'un seul élément piézo-électrique émetteur/récepteur. L'émission se fait durant un certain temps, puis elle est interrompue, et suivie de la réception des signaux réfléchis. La fréquence des alternances émission/réception est calculée en fonction de la profondeur à explorer, c'est-à-dire du diamètre du vaisseau.

Pour appréhender la profondeur à explorer et la vélocité, du sang (ou autre fluide), on applique la sonde sur la paroi de l'organe puis on émet des ondes ultrasonores à une distance d'exploration de plus en plus grande. Tant que ces ondes ne pénètrent pas à l'intérieur du vaisseau, elles sont réfléchies par des obstacles fixes, et aucune variation de fréquence n'est constatée. La plage des distances d'exploration pour lesquelles une variation de fréquence est observée permet donc d'appréhender par différence le diamètre intérieur du vaisseau sanguin. On comprend dans ce cas l'importance de la précision avec laquelle on applique la sonde sur l'organe. En effet, si celle-ci n'est pas parfaitement centrée, la profondeur d'exploration mesurée ne correspondra pas au diamètre véritable du vaisseau.

Pour mesurer la vélocité du sang dans un vaisseau sanguin à l'aide de telles sondes périvasculaires ultrasonores à effet Doppler, on est obligé, à chaque fois que l'on veut faire une mesure, de procéder à l'application de la sonde sur l'organe à explorer. A la fin de la mesure, on enlève cette sonde. Or, l'inventeur a déterminé que ces manipulations des sondes sont mal commodes, gênantes, et relativement longues à effectuer. De plus, les disques piézo-électriques employés dans de telles sondes sont de faible épaisseur (de l'ordre de quelques dixièmes de millimètres) et donc relativement fragiles. Il s'agit donc d'éviter au maximum les manipulations des sondes afin de prévenir la détérioration des disques piézo-électriques.

On connaît également de nombreux types de sondes endoscopiques (demande de brevet français 2 461 486, 2 507 075, 2 543 817 ...). Ces sondes ne sont pas destinées à être appliquées au contact d'un organe à explorer. Elles n'ont donc pas à être implantables ni extractibles. De plus, ces sondes ne sont pas applicables à la débitmétrie par effet Doppler. Au contraire, l'invention ne concerne pas le domaine technique des sondes endoscopiques pour lesquelles les problèmes posés et les moyens mis en oeuvre sont différents de ceux des sondes directement appliquées contre la paroi d' organe à explorer.

La présente invention remédie à ces inconvénients en proposant une sonde ultrasonore implantable dans le corps humain ou animal destinée à être appliquée au contact d'une paroi d'un organe à sonder.

Dans le cadre de l'invention, une sonde est dite implantable lorsqu'on peut l'appliquer contre une paroi d' un organe à explorer à l'intérieur d'un corps humain ou animal et la laisser appliquée à l'intérieur de ce corps aussi longtemps qu'on le désire, ou au moins pendant un laps de temps nettement supérieur à la durée de l'examen par cette sonde, sans qu'elle crée des phénomènes de rejets ou autres traumatismes dans ce corps.

Cependant, plusieurs problèmes s'opposent à la réalisation d'une telle sonde implantable, à savoir notamment :

- le choix d'un matériau implantable et transparent aux ultrasons. En effet, les matériaux implantables connus sont généralement opaques aux ultrasons,
- l'étanchéité et l'isolation électrique. En effet, pour être implantable, une sonde doit être parfaitement étanche et isolée électriquement et les fluides environnants ne doivent en aucun cas pouvoir pénétrer à l'intérieur de la sonde, même à la longue, et provoquer des dégâts tels que court-circuits, fuites de courant électrique, corrosion, ...
- l'extraction de la sonde doit pouvoir être effectuée facilement,
- la sonde doit être de faibles dimensions. Or les ondes ultrasonores doivent être émises et reçues avec une certaine incidence dans le fluide, ce qui entraîne que les moyens piézo-électriques émetteurs/récepteurs doivent être inclinés par rapport à la direction de déplacement de ce fluide, classiquement de 45° dans les sondes à effet Doppler connues. Ce phénomène implique des dimensions minimales de la sonde en hauteur.

- le problème du couplage acoustique des moyens émetteurs/récepteurs avec ce matériau.
- le problème de la fixation de la sonde. En effet, la sonde ne doit pas être susceptible de se déplacer, et doit rester parfaitement centrée par rapport à l'organe, notamment lorsqu'il s'agit d'un vaisseau.

L'invention vise à remédier aux inconvénients précités, et pour ce faire, elle propose une sonde émettrice/réceptrice d'ultrasons implantable dans le corps humain ou animal, destinée à être appliquée au contact d'une paroi d'un organe pour permettre une exploration à l'intérieur de l'organe par ultrasons -notamment par effet Doppler- comportant des moyens supports de moyens transducteurs piézo-électriques émetteurs/récepteurs, et un enrobage recouvrant les moyens émetteurs/récepteurs et au moins la partie des moyens supports opposée à l'organe, la face émettrice/réceptrice des moyens émetteurs/récepteurs étant associée en contact avec une face des moyens supports qui sont intercalés entre les moyens émetteurs/récepteurs et l'organe, la sonde comportant également des moyens de liaison avec l'extérieur du corps véhiculant les informations émises/reçues par/sur la sonde, les moyens supports comportant une face extérieure d'application sur le vaisseau ou une paroi d'un organe et caractérisée en ce que les moyens supports sont constitués d'un élastomère silicone non chargé et en ce que l'enrobage est constitué d'un élastomère silicone chargé opaque aux ultrasons, les moyens supports et l'enrobage étant configurés et formés de façon à permettre l'extraction de la sonde du corps sans intervention chirurgicale complexe, notamment par simple traction de l'extérieur sur les moyens de liaison. La sonde comporte de préférence des moyens d'étanchéité et d'isolation assurant l'étanchéité et l'isolation électrique totales de la sonde vis-à-vis des fluides environnants. La surface extérieure de l'enrobage est lissée et sensiblement en forme de goutte. Selon un mode de réalisation, la sonde comporte des moyens d'association rigide de cette sonde sur l'organe à explorer ; des moyens d'adaptation de l'impédance acoustique entre les moyens émetteurs/récepteurs et les moyens supports ; des moyens de protection des moyens émetteurs/récepteurs aux moyens supports.

Une telle sonde est donc véritablement implantable sur un animal ou un être humain et peut être appliquée par exemple à la mesure répétée de la vélocité sanguine et donc du débit sanguin dans un vaisseau (veine, artère ou autre) de l'animal ou de l'être humain selon les besoins. On pourra ainsi prévoir l'implantation d'une telle sonde chez les sujets pour lesquels on doit appréhender régulièrement le débit sanguin, par exemple pour suivre des problèmes ou maladies cardio-vasculaires, ou autres. Une autre application de l'invention peut être de mesurer l'épaisseur d'une paroi.

On remarquera, de plus, que l'inventeur a déterminé notamment que non seulement les élastomères silicones non chargés sont bon conducteurs des ultrasons, mais de plus que la célérité des ultrasons est plus faible dans ces élastomères silicones que dans les matériaux utilisés pour la fabrication des sondes connues, tels que le plexiglas (marque déposée) ou l'altuglas (marque déposée). De ce fait, les moyens émetteurs/récepteurs d'une sonde selon l'invention sont moins inclinés par rapport à la direction de propagation du fluide, ce qui permet d'en réduire la hauteur.

L'invention sera mieux comprise à la lecture de la description suivante d'un mode de réalisation d'une sonde ultrasonore à effet Doppler débitmétrique, donné à titre d'exemple non limitatif, et qui fait référence aux figures annexées, dans lesquelles :
- la figure 1 est une vue schématique de profil d'une sonde selon l'invention;
- la figure 2 est une vue schématique de face (de gauche par rapport à la figure 1) d'un autre mode de réalisation d'une sonde selon l'invention.

Une sonde à ultrasons comprend des moyens 2 d'investigations de l'organe à explorer, et des moyens 3 de liaison électrique avec l'extérieur.

Les moyens 3 de liaison comportent un câble 4 de sortie et une prise de connexion électrique 5 à son extrémité opppposée de la sonde, permettant de brancher la sonde sur des appareils extérieurs (source d'énergie, moyens d'analyse ...) non représentés.

Les moyens 2 d'investigations comportent, de façon connue en soi, des moyens transducteurs piézo-électriques 6 émetteurs/récepteurs d'ondes ultrasonores. Ces moyens transducteurs émetteurs/récepteurs 6 se présentent généralement sous la forme d'un disque de dimensions variables selon la fréquence propre désirée et sont connectés électriquement au câble 4. Une face du disque constitue alors une face utile émettrice/réceptrice 7 des moyens piézo-électriques 6 émetteurs/récepteurs par laquelle les ondes ultrasonores permettant l'exploration de l'organe sont émises/reçues. Les moyens transducteurs piézo-électriques émetteurs/récepteurs 6 transforment de l'énergie électrique en ondes ultrasonores qu'ils émettent et réciproquement transforment les ondes ultrasonores qu'il reçoivent en courant électrique.

Une sonde 1 selon l'invention comprend des moyens supports 8 en matériau implantable bon conducteur des ultrasons. Ce matériau est un élastomère silicone non chargé, qui s'avère être bon conducteur des ultrasons. Les moyens émetteurs/récepteurs piézo-électriques 6 sont associés aux moyens supports 8. La face 7 émettrice/réceptrice de ces moyens émetteurs/récepteurs 6 est associée en contact avec une face 16 des moyens supports 8 par des moyens 9 d'association rigide des moyens émetteurs/récepteurs 6 aux moyens supports 8. Un enrobage 10 de matériau implantable opaque aux ultrasons recouvre à la fois au moins la partie 11 des moyens supports 8 opposée à l'organe à explorer 15, les moyens émetteurs/récepteurs 6, l'extrémité 12 du câble à laquelle les moyens émetteurs/récepteurs 6 sont connectés et la connexion 13 entre le câble 4 et les moyens émetteurs/récepteur 6.

Une sonde 1 comporte de préférence moyens d'étanchéité assurant l'étanchéité totale de la sonde 1 vis-à-vis des fluides environnants, sans perturber ses caractéristiques mécaniques et acoustiques, et des moyens d'isolation électrique assurant l'isolation électrique parfaite des conducteurs 20, 21, de la connexion 13 et du disque piézo-électrique 6 vis-à-vis de l'extérieur.

La fonction de l'enrobage 10 est de former un écran opaque aux ultrasons qui pourraient être émis/reçus dans des directions sensiblement distinctes de la normale 14 à cette face 7 émettrice/réceptrice des moyens émetteurs/récepteurs 6, de consolider la connexion des moyens émetteurs/récepteurs 6 au câble 4, et d'assurer la protection et le maintien de ces moyens émetteurs/récepteurs 6 sur les moyens supports 8, tout en étant implantable. L'enrobage 10 peut être en adhésif silicone.

De plus, une étanchéité parfaite peut être obtenue en enfilant une gaine mince en élastomère de silicone non chargé sur la sonde ainsi enrobée d'adhésif silicone immédiatement après le dépôt de cet adhésif. Cette gaine épouse la forme générale de la sonde ; la fermeture de son extrémité s'effectue en la maintenant pincée pendant le durcissement de l'adhésif. L'utilisation d'un micro-doigtier en élastomère de silicone non chargé est préférable. Dans les deux cas, la paroi placée devant la face de sondage 17 ne gêne pas le passage des ultrasons. L'étanchéité sera de toutes façons d'autant meilleure que le nombre des surfaces de contact des différents éléments de la sonde débouchant à l'extérieur en une ligne de contact, est minimum.

C'est pourquoi dans une sonde selon l'invention, les moyens d'étanchéité et d'isolation électrique (non représentés) sont ensemble constitués d'une gaine mince de matériau implantable transparent aux ultrasons, notamment en élastomère silicone non chargé, qui recouvre, en en épousant la forme extérieure, les moyens supports 8, l'enrobage 10 en adhésif silicone, et tout ou partie - notamment l'extrémité 12 proche des moyens émetteurs/récepteurs 6- du câble 4.

Dans les modes de réalisation de l'invention, représentés sur les figures, les moyens supports 8 comportent une face extérieure 17 d'application sur l'organe 15 à explorer formant la face d'émission/réception de la sonde 1, les ondes ultrasonores étant émises/reçues de/sur cette face extérieure 17 vers/en provenance de l'organe à explorer 15, et une face intérieure opposée 16 en contact avec les moyens émetteurs/récepteurs 6 de sorte que les ondes ultrasonores émises par la face émettrice/réceptrice 7 des moyens émetteurs/récepteurs 6, au niveau de la face intérieure opposée 16 traversent les moyens supports 8 puis sortent de la sonde par la face extérieure 17 et inversement, les ondes en provenance de l'organe pénètrent dans la sonde par la face extérieure 17, traversent les moyens supports 8 pour être captées par la face émettrice/réceptrice 7 des moyens émetteurs/récepteurs 6 à la sortie de la face intérieure 16.

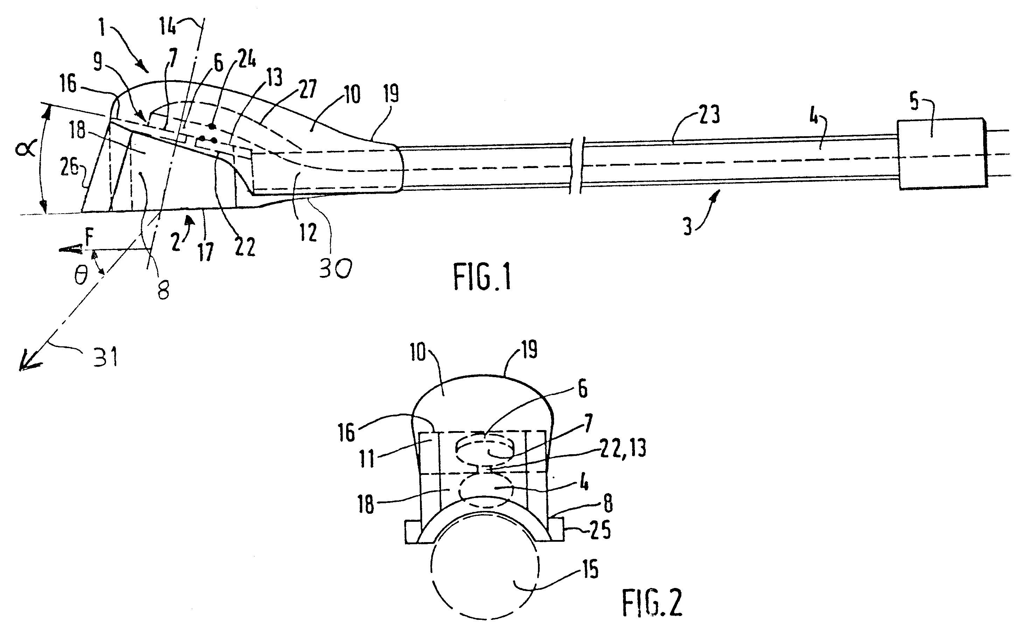

Les ondes ultrasonores doivent être émises sous une certaine incidence par rapport à la direction de déplacement du fluide dont on mesure la vitesse (matérialisée par la flèche F sur la figure 1). Cet angle d'incidence est avantageusement de l'ordre de 60° pour une sonde ultrasonore à 8MHZ. Par conséquent, la face émettrice/réceptrice 7 doit aussi être inclinée. Cependant, dans le calcul de cette inclinaison il faut tenir compte de la loi de Descartes et des différentes célérités des ondes ultrasonores dans l'élastomère silicone et dans le fluide considéré. Ainsi, dans une sonde 1 selon un mode de réalisation, la face intérieure opposée 16 des moyens supports 8 est inclinée d'un angle $\alpha$ notamment de l'ordre de 20° par rapport à la face extérieure 17 de ces moyens supports 8 de sorte que les ondes ultrasonores pénètrent une direction 31 dans l'organe à explorer sous un angle $\theta$ approprié, notamment de 60° par rapport à la direction de déplacement du fluide dont on veut mesurer la vitesse. La face antérieure 26 des moyens supports 8 est orientée de manière à être parallèle à ou à diverger de la normale 14 dans le sens de l'émission des ondes, ceci assurant qu'aucun rayon émis ou reçu ne risque de frapper contre cette paroi.

On prévoit avantageusement que l'enrobage 10 recouvre la majeure partie des moyens supports 8, en ne laissant libre que la face extérieure 17 d'application sur l'organe 15 à explorer, afin que cet enrobage fasse écran opaque aux ultrasons de la façon la plus efficace. En effet, plus important sera le recouvrement de cet enrobage 10, moins on aura d'ultrasons parasites inutiles émis ou reçus, et meilleure sera l'exactitude des renseignements fournis par la sonde 1. Dans les modes de réalisation de l'invention, représentés sur les figures, l'enrobage 10 ne recouvre pas entièrement les flancs 18 des moyens supports 8, et ce pour plus de clarté. Il est cependant préférable que l'enrobage 10 recouvre ces flancs 18 et plus généralement, l'enrobage 10 recouvre les moyens d'investigations 2, sauf la face extérieure d'application d'émission/réception 17. Ainsi, en plus d'un meilleur isolement acoustique, l'étanchéité est mieux assurée. Il est avantageux que les flancs 18 soient inclinés, et forment avec la face extérieure 17 un angle inférieur à 90°. Ces flancs 18 peuvent même être tangents au disque piézo-électrique 6. La face antérieure 26 peut également être tangente au disque 6, et former avec la face 17 un angle nettement inférieur à 90° La paroi antérieure peut être semi-circulaire inclinée.

La surface extérieure 19 de l'enrobage 10 est avantageusement lissée et sensiblement en forme de goutte, s'élargissant progressivement et continûment à partir de l'extrémité 12 du câble 4 à laquelle les moyens émetteurs/récepteurs 6 sont connectés, pour enrober les moyens émetteurs/récepteurs 6, la connexion électrique 13 et les moyens supports 8 sauf la face extérieure 17 d'application sur l'organe 15 à explorer, de façon à faciliter son extraction éventuelle sans intervention chirurgicale complexe.

La sonde 1 selon l'invention comporte avantageusement des moyens d'association rigide (non représentés) à l'organe 15 à explorer évitant tout déplacement intempestif ultérieur de la sonde 1 lorsqu'elle est implantée mais permettant toutefois son extraction ultérieure. Par exemple, ces moyens d'association rigide sur l'organe 15 sont constitués d'un morceau de matière synthétique tissé, implantable et apte à s'intégrer à l'organe 15, de faible épaisseur, associé rigidement, mais de façon amovible sous l'effet d'une traction, notamment par collage, à la sonde de façon à être tangent à la face d'émission/réception 17 de la sonde, un évidement étant pratiqué dans ce morceau de matière synthétique en regard de la face d'émission/réception 17 pour autoriser le passage des ondes ultrasonores.

De plus, l'impédance acoustique Z1 des moyens émetteurs/récepteurs piézo-électriques 6, généralement en céramique, est très différente de celle Z2 des moyens supports 8 en élastomère silicone. Par conséquent, le sondes ultrasonores se trouvent fortement perturbées à leur passage de ces moyens émetteurs/récepteurs 6 dans les moyens supports 8, par des phénomènes connus, notamment de réflexions parasites. C'est pourquoi une sonde 1 selon l'invention comporte avantageusement des moyens d'adaptation de l'impédance acoustique entre les moyens émetteurs/récepteurs 6 et les moyens supports 8 permettant notamment d'éviter les réflexions parasites au passage des ondes entre les moyens supports 8 et les moyens émetteurs/récepteurs 6. Ces moyens d'adaptation d'impédance sont constitués d'une couche d'un matériau, qui est de' préférence implantable, c'est-à-dire sans danger pour le corps humain ou animal, interposé entre les moyens émetteurs/récepteurs 6 et les moyens supports 8. L'impédance acoustique $Z_3$ de ces moyens d'adaptation d'impédance est comprise entre les impédances acoustiques $Z_1$ et $Z_2$ des moyens émetteurs/récepteurs 6 et des moyens supports 8, de préférence voisine de la moyenne géométrique de ces impédances.

Classiquement, l'impédance acoustique $Z_1$ des moyens émetteurs/récepteurs piézo-électriques 6 est de l'ordre de 30 Pa.s/m, tandis que celle $Z_2$ des moyens supports 8 en élastomère silicone est de l'ordre de 1 Pa.s/m. Ainsi, les moyens d'adaptation d'impédance acoustique auront avantageusement une impédance acoustique comprise entre 3 et 10 Pa.s/m, notamment de l'ordre de 5 Pa.s/m.

L'inventeur a déterminé que les peintures pour galvanisation à froid offrent les qualités requises pour réaliser ces moyens d'adaptation d'impédance acoustique : elles peuvent être pulvérisées en une mince couche, elles sont sans danger pour l'organisme et ont une impédance acoustique satisfaisante. C'est pourquoi les moyens d'adaptation d'impédance acoustique sont de préférence constitués par une mince couche de mélange pulvérisé sur la face émettrice/réceptrice 7 des moyens émetteurs/récepteurs 6. La mince couche pulvérisée est une couche de peinture du type pour galvanisation à froid, par exemple contenant du zinc et des résines epoxy. Ce mélange se pulvérise bien et n'est pas conducteur de l'électricité, ce qui évite des problèmes de court-circuit ou faux-contact avec les connexions électriques 13. Il est à noter que cette couche d'adaptation d'impédance abaisse la fréquence propre des moyens émetteurs/récepteurs 6, qu'il faudra adapter en conséquence.

Les moyens transducteurs émetteurs/récepteurs 6 sont avantageusement constitués d'un disque de céramique piézo-électrique, de préférence en zirconate de Baryum. Pour une fréquence d'émission de la sonde de l'ordre de 8MHZ, le diamètre de ce disque est d'environ 4 mm et son épaisseur est de 0,25 mm environ. Ses faces sont argentées pour permettre la connexion électrique 13, notamment par soudure, des conducteurs du câble 4 à ces faces.

Afin d'éviter la détérioration de ce disque lors de l'application de la sonde 1 sur l'organe 15, lors des manipulations, ou lors de son extraction la sonde 1 comporte avantageusement des moyens de protection 27 dont la fonction est de protéger les moyens émetteurs/récepteurs 6 et/ou les moyens 9 d'association rigide de ces moyens émetteurs/récepteurs 6 aux moyens supports 8 afin d'éviter la détérioration des moyens émetteurs/récepteurs 6 et/ou de l'association rigide aux moyens supports 8.

Ces moyens de protection 27 sont avantageusement constitués d'un élément rigide disposé par dessus le disque piézo-électrique 6, par exemple en forme de capot, de préférence sans contact avec ce disque.

D'autres formes de réalisation peuvent être envisagées, notamment dépôt d'une goutte de matière plastique 27, assez rigide pour renforcer le disque piézo-électrique 6, notamment dans sa partie arrière contenant la connexion 13. Cette goutte, de forme basse, doit avoir son maximum d'épaisseur en regard du bord arrière du disque 6 (point de fragilité de la sonde 1) et ne présente aucune surface parallèle à ce disque 6. Une résine synthétique telle que polyester semble tout indiquée de par ses qualités mécaniques acoustiques et biocompatibles. Une structure rigide expansée à micro-bulles non communicantes serait parfaite. L'enrobage 10 général peut être un adhésif silicone assurant ainsi de plus une excellente étanchéité.

Les moyens 9 d'association rigide des moyens émetteurs/récepteurs 6 aux moyens supports 8 sont constitués d'une mince couche de colle, notamment cyanocrylate implantable (de type B), interposée entre la face intérieure 16 des moyens supports 8 et les moyens d'adaptation d'impédance, ou la face émettrice/réceptrice 7 des moyens émetteurs/récepteurs.

Cette couche de colle doit être de la plus faible épaisseur possible afin de ne pas modifier trop la fréquence des ondes ultrasonores, et avoir une impédance acoustique intermédiaire entre celle $Z_3$ des moyens d'adaptation d'impédance et celle $Z_2$ des moyens supports 8, de préférence voisine de la moyenne géométrique de ces impédances $Z_3$ et $Z_2$.

Le câble 4 est habituellement du type coaxial mais peut être bifilaire et torsadé, ce qui s'avère avantageux en pratique, notamment pour les appareils externes dont l'entrée est symétrique. Ses conducteurs sont de préférence en cuivre nu divisé, ou argentés, et sont soudés sur les faces argentées des moyens émetteurs/récepteurs 6. Le câble 4 comporte notamment et de préférence un conducteur soudé sur la face 7 émettrice/réceptrice, et un conducteur soudé sur la face opposée 24 à la face émettrice/réceptrice 7.

Dans ce dernier cas, la face 16 intérieure des moyens support 8 en contact avec les moyens émetteurs/récepteurs 6 comporte avantageusement un évidement 22 permettant le passage des conducteurs; ceux-ci peuvent être associés rigidement aux moyens supports 8, par exemple par collage, à l'aide d'une goutte de colle cyanocrylate, à l'intérieur de l'évidement 22, pour assurer le maintien de la connexion électrique 13 lors des manipulations de la sonde 1. Le câble 4 est protégé sur toute sa longueur par une gaine 23 en matériau implantable, notamment en élastomère silicone chargé ou non. La prise de connexion 5 est de préférence miniature, réversible à trois broches à contacts dorés.

Avantageusement, la gaine 23 recouvre également l'enrobage 10 et/ou les moyens supports 8 pour former les moyens d'étanchéité ou d'isolation électrique de la sonde 1. Dans ce cas, la sonde 1 ne comporte plus aucune ligne de contact extérieure et est parfaitement étanche et isolée électriquement.

Les moyens supports 8 sont constitués avantageusement d'un bloc d'élastomère silicone non chargé, ou autre matériau implantable bon conducteur des ultrasons, formant une portion de cylindre ou de cône, dont les bases constituent les faces intérieure 16 et extérieure 17 de ces moyens supports 8.

En figure 1, on a représenté un premier mode de réalisation de l'invention pour lequel la face extérieure 17 d'application des moyens supports 8 sur l'organe 15 à explorer est plane. Cependant, afin de permettre une meilleure adaptation de la sonde à l'organe 15 à explorer, la face extérieure 17 d'application a préférentiellement une forme courbe correspondant à la surface extérieure de cet organe 15. En particulier, dans le cas ou l'organe 15 est un vaisseau sanguin, la face extérieure 17 est cylindrique concave, de façon à être adaptée à la forme extérieure de l'organe 15 à explorer (figure 2).

Deux petits ailerons latéraux 25 facilitent la fixation de la sonde 1 sur l'organe 15, ceci par les moyens d'association rigide sur l'organe à explorer, par exemple un fil résorbable ou de la manière précédemment mentionnée. L'angle d'enveloppement du vaisseau ne doit pas être excessif afin de s'accommoder d'une certaine plage de diamètre des vaisseaux à explorer.

La face 17 d'émission/réception de la sonde 1 doit de préférence faire saillie vers le bas par rapport à l'enrobage 10, de façon à être correctement en contact avec la paroi de l'organe 15. Cette face 17 est inclinée à sa partie arrière 30 pour rejoindre progressivement l'enrobage 10.

L'élastomère silicone est un matériau relativement difficile à usiner. C'est pourquoi on aura avantage à utiliser une technique de découpage ou de moulage des moyens supports 8 dans un bloc d'élastomère silicone non chargé. Dans le cas du découpage, les faces intérieure 16 et extérieure 17 des moyens supports 8 sont polygonales. La forme de découpe ou de moulage est prévue pour se rapprocher au maximum de la sphère, en étant la plus ronde possible afin de faciliter l'installation et/ou l'extraction de la sonde. En effet, il sera d'autant plus facile de donner une forme de goutte à la surface extérieure 19 de l'enrobage 10 que les moyens supports 8 auront eux-mêmes une forme ronde.

Dans un procédé de fabrication d'une sonde 1 selon l'invention, on fabrique les moyens supports 8, notamment par découpage ou moulage d'un bloc d'élastomère silicone, en leur donnant la forme appropriée, puis on associe rigidement, notamment par collage, à ces moyens supports 8 les moyens émetteurs/récepteurs piézo-électriques 6 que l'on aura préalablement soudés au câble de sortie 4, puis on teste les caractéristiques de la sonde (notamment on mesure la fréquence), et on place, lorsque ceux-ci sont prévus, les moyens de protection 27, puis un enrobage 10 recouvrant les moyens émetteurs/récepteurs 6, la connexion 13 avec le câble de sortie 4, l'extrémité 12 correspondante de ce câble et les moyens supports 8 sauf la face extérieure 17 d'application sur l'organe 15 à explorer et on place lorsque ceux-ci sont prévus les moyens d'étanchéité et/ou d'isolation électrique.

Lorsque les moyens supports 8 comportent une face extérieure 17 concave et/ou des ailerons 25, sa fabrication par moulage est pratiquement imposée. De toute façon, cette réalisation par moulage est avantageuse car elle permet d'une part la réduction des coûts de fabrication des moyens supports 8, et d'autre part, une fabrication plus régulière de ces moyens supports 8, qui ont des performances moins dispersées, ce qui permet de rejeter moins de sondes en cours de fabrication.

De préférence, on mesure et on note une première fois la fréquence propre Fo des moyens émetteurs/récepteurs 6, connectés au câble 4, puis on dépose des moyens d'adaptation d'impédance sur les moyens émetteurs/récepteurs 6, notamment sur leur face émittrice/réceptrice 7. Avant de les associer aux moyens supports 8, on mesure une seconde fois la fréquence propres Fo' des moyens émetteurs/récepteurs piézo-électriques 6 : on doit alors trouver un écart de fréquence $F = Fo-Fo'$ ayant une certaine valeur qu'il importe de respecter. Dans le cas d'une valeur trouvée hors tolérances, il faut retoucher cette couche déposée : ceci s'obtient par pulvérisation complémentaire, grattage ou élimination complète par solvant afin de recommencer entièrement la pulvérisation.

Dans un procédé de fabrication d'une sonde selon l'invention, il est nécessaire de choisir des moyens émetteurs/récepteurs qui ont une fréquence propre supérieure à la fréquence désirée pour les ondes ultrasonores d'investigations. En effet, les moyens d'adaptation d'impédance, ainsi que les moyens 9 d'association rigide des moyens émetteurs/récepteurs 6 sur les moyens supports 8 abaissent la fréquence propre des moyens émetteurs/récepteurs 6. D'ailleurs, on ajuste la fréquence en pulvérisant une couche adéquate d'adaptation d'impédance et en associant les moyens émetteurs/récepteurs 6 sur les moyens supports 8. C'est d'ailleurs la variation de la fréquence qui indiquera l'épaisseur de la couche déposée, et donc les propriétés acoustiques de cette couche. Il faudra tenir compte également de l'épaisseur de colle utilisée dans le choix des moyens émetteurs/ récepteurs.

Après association aux moyens supports 8, on mesure une troisième fois la fréquence Fo'' tout en observant l'allure générale de la courbe d'impédance Z. Ce contrôle est passif, c'est-à-dire que l'on n'a plus d'action possible pour corriger les caractéristiques de la sonde obtenue, contrairement à la seconde mesure de fréquence Fo'.

Avant de réaliser l'enrobage 10, il est souhaitable d'effectuer un essai utilisant la sonde 1 en temps qu'émetteur/récepteur d'ultrasons à l'aide d'un échographe en mode A. La sonde est alors plongée dans un récipient contenant de l'eau distillée et une cible fixe. L'amplitude et la forme de l'écho visualisé par l'échographe complète l'information donnée par la mesure de Fo''. Ce contrôle échoigraphique est capital quant à l'efficacité de la sonde 1 en cours de fabrication : il peut faire rejeter une sonde ayant satisfait à la mesure de Fo'' à partir de la courbe d'impédance Z déjà décrite.

A titre d'exemple, on choisira un disque piézoélectrique d'environ 4 mm de diamètre et 0,25 mm d'épaisseur qui aura une fréquence propre supérieure à 8MHZ. Par exemple, en supposant que cette fréquence propre soit de 8,41MHZ (mesurée la première fois), il faudrait l'abaisser d'environ 0,25MHZ par dépôt de la couche d'adaptation d'impédance, en déposant une couche comprise entre environ 10 et 15 um. Ainsi, la fréquence d'émission des ondes ultrasonores de la sonde 1 terminée sera voisine de 8MHZ.

Une sonde 1 selon l'invention est en particulier applicable à la mesure répétée de la vélocité et du débit du sang dans un vaisseau sanguin de l'être humain selon les besoins.

## Revendications

**1.** Sonde émettrice/réceptrice d'ultrasons implantable dans le corps humain ou animal, destinée à être appliquée au contact d'une paroi d'un organe (15) pour permettre une exploration à l'intérieur de l'organe (15) par ultrasons - notamment par effet Doppler-, comportant des moyens supports (8) de moyens transducteurs piézo-électriques émetteurs/récepteurs (6), et un enrobage (10) recouvrant les moyens émetteurs/récepteurs (6) et au moins la partie (11) des moyens supports (8) opposée à l'organe (15), la face (7) émettrice/réceptrice des moyens émetteurs/récepteurs (6) étant associée en contact avec une face (16) des moyens supports (8) qui sont intercalés entre les moyens émetteurs/récepteurs (6) et l'organe (15), la sonde (1) comportant également des moyens (3) de liaison avec l'extérieur du corps véhiculant les informations émises/reçues par/sur la sonde (1), les moyens supports (8) comportant une face extérieure (17) d'application sur le vaisseau (15) ou une paroi d'un organe caractérisée en ce que les moyens supports (8) sont constitués d'un élastomère silicone non chargé et en ce que l'enrobage (10) est constitué d'un élastomère silicone chargé opaque aux ultrasons, les moyens supports (8) et l'enrobage (10) étant configurés et formés de façon à permettre l'extraction de la sonde (1) du corps sans intervention chirurgicale complexe, notamment par simple traction de l'extérieur sur les moyens (3) de liaison.

**2.** Sonde selon la revendication 1 caractérisée en ce que la surface extérieure (19) de l'enrobage (10) est lissée et est sensiblement en forme de goutte, s'élargissant progressivement et continûment à partir de l'extrémité (12) du câble (4) à laquelle les moyens émetteurs/récepteurs (6) sont connectés, pour enrober les moyens émetteurs/récepteurs (6), la connexion électrique (13) et tout ou partie des moyens supports (8).

**3.** Sonde selon l'une quelconque des revendications 1 et 2 comportant des moyens d'étanchéité assurant l'étanchéité totale de la sonde (1) vis-à-vis des fluides environnants, sans perturber ses caractéristiques mécaniques et acoustiques et des moyens isolation électrique assurant l'isolation électrique parfaite vis-à-vis de l'extérieur des conducteurs, de la connexion (13) et du disque piézo-électrique (6), caractérisée en ce que les moyens d'étanchéité et d'isolation électrique sont ensemble constitués d'une gaine mince de matériau implantable transparent aux ultrasons, notamment en élastomère silicone non chargé, qui recouvre, en épousant leur forme extérieure, les moyens supports (8), l'enrobage (10) en adhésif silicone, et tout ou partie -notamment l'extrémité (12) proche des moyens émetteurs/récepteurs (6)- du câble (4).

4. Sonde selon l'une quelconque des revendications 1 à 3, caractérisée en ce qu'elle comporte des moyens d'adaptation de l'impédance acoustique entre les moyens émetteurs/récepteurs (6) et les moyens supports (8), et en ce que ces moyens d'adaptation d'impédance sont constitués par une mince couche de mélange pulvérisée sur la face émettrice/réceptrice (7) des moyens émetteurs/récepteurs (6), permettant notamment d'éviter les réflexions parasites au passage des ondes entre les moyens supports (8) et les moyens émetteurs/récepteurs.

5. Sonde selon la revendication 4 caractérisée en ce que la mince couche pulvérisée est une couche de peinture du type pour galvanisation à froid, notamment contenant du zinc et des résines epoxy.

6. Sonde selon l'une quelconque des revendications 1 à 5, caractérisée en ce qu'elle comporte des moyens de protection (27) dont la fonction est de protéger les moyens émetteurs/récepteurs (6) et/ou les moyens (9) d'association rigide de ces moyens émetteurs/récepteurs (6) aux moyens supports (8) afin d'éviter la détérioration des moyens émetteurs/récepteur (6) et/ou de l'association rigide aux moyens supports (8) lorsque l'on manipule la sonde (1), et en ce que les moyens de protection (27) sont constitués d'une goutte de matière plastique rigide (27) qui a son maximum d'épaisseur en regard du bord arrière du disque piézo-électrique (6) et qui ne présente aucune surface parallèle du disque (6).

7. Sonde selon l'une quelconque des revendications 1 à 6, caractérisée en ce que les moyens supports (8) sont constitués avantageusement d'un bloc d'élastomère silicone non chargé bon conducteur des ultrasons, formant une portion de cylindre ou de cône dont les bases constituent les faces intérieure (16) et extérieure (17) de ces moyens supports (8), et en ce que la face antérieure (26) et/ou les flancs (18) des moyens supports (8) sont inclinés et forment avec la face extérieure (17) un angle inférieur à 90°.

8. Sonde selon l'une quelconque des revendications 1 à 7, caractérisée en ce que la face antérieure (26) et/ou les flancs (18) des moyens supports (8) sont tangents au disque piézo-électrique.

9. Sonde selon l'une quelconque des revendications 1 à 8, caractérisée en ce qu'elle comporte des moyens d'association rigide sur l'organe (15) à explorer, évitant tout déplacement intempestif ultérieur de cette sonde (1), et en ce qu'elle comporte deux ailerons latéraux (25) qui facilitent la fixation de la sonde (1) sur l'organe (15) par les moyens d'association rigide sur l'organe (15), notamment du fil résorbable.

10. Sonde selon l'une quelconque des revendications 1 à 9, caractérisée en ce que la face intérieure opposée (16) des moyens supports (8) est inclinée d'un angle $\alpha$ notamment de l'ordre de 20° par rapport à la face extérieure (17) de ces moyens supports (8) de sorte que les ondes ultrasonores pénètrent dans l'organe à explorer sous un angle $\theta$ approprié, notamment de 60°, par rapport à la direction de déplacement du fluide dont on veut mesurer la vitesse.

11. Sonde selon l'une quelconque des revendications 1 à 10, caractérisée en ce que la face (7) d'émission/réception fait saillie vers le bas par rapport à l'enrobage (10), et en ce que la partie arrière (30) de cette face (7) est inclinée pour rejoindre progressivement l'enrobage (10).

**Claims**

1. Ultrasonic transmitter/receiver probe which can be implanted into the human or animal body and is intended to be applied by contact to the surface of an organ (15) in order to permit exploration of the interior of the organ (15) by ultrasonics - more particularly by way of the Doppler Effect - comprising support means (8), transmitter/receiver piezo-electric transducer means (6), and a casing covering the transmitter/receiver means (6), and at least the part (11) of the support means (8) opposite the organ (15), the transmitter/receiver surface (7) of the transmitter/receiver means (6) being in contact with a surface (16) of the support means (8) which are located between the transmitter/receiver means (6) and the organ (15), the probe (1) also having means (3) of connection with the exterior of the body conveying the information transmitted by/received from the probe (1), the support means (8) consisting of an external surface (17) for application on the vessel (15) or organ surface, characterised in that the support means (8) are made of an uncharged silicon elastomer and that the casing (10) consists of a charged silicon elastomer which is impervious to ultasound, the support means (8) and casing (10) being arranged and shaped in such a way as to permit extracting of the probe (1) from the body without the need for complex surgery, more particularly by way of simple traction on the exterior of the connection means (3).

2. Probe in accordance with claim 1 characterised in that the external surface (19) of the casing (10) is smooth and slightly drop-shaped becoming progressively broader and continuous from the end (12) of the cable (4) to which the transmitter/receiver means (6) are connected so as to cover the transmitter/ receiver means (6), the electrical connection (13) and all or part of the support means (8).

3. Probe according to any one of claims 1 and 2 comprising sealing means to ensure total sealing of the probe (1) vis-a-vis ambient fluids without disturbing its mechanical and acoustic characteristics and means of electrical insulation providing perfect electrical isolation vis-a-vis the exterior of the conductors, connection (3) and the piezo-electric disk (6), characterised in that the sealing and electrical insulation means consist of a thin sheath of implantable material which is permeable for ultrasound, more particularly an uncharged silicon elastomer covering, moulding to their external shape, the support means (8), the casing (10) in silicon adhesive, and all or part - more particularly the end (12) close to the transmitters/receivers (6) - of the cable (4).

4. Probe according to any one of claims 1 to 3, characterised in that it includes means of adapting the acoustic impedance between the transmitters/receivers (6) and the support means, and in that these means of adapting the impedance are constituted by a thin layer of pulverized mixture on the transmitter/receiver surface (7) of the transmitters/receivers (6), allowing parasitic reflection to be avoided as the waves pass between the support means (8) and the transmitter/receiver means.

5. Probe according to claim 4 characterised in that the thin pulverized layer is a layer of paint of the type used for dry galvanizing, more particularly containing zinc and epoxy resins.

6. Probe according to any one of claims 1 to 5 characterised in that it comprises protection means (27) the function of which is to protect the transmitter/receiver means (6) and/or the means (9) of firmly attaching these transmitter/receiver means (6) to the support means (8) so as to prevent deterioration of the transmitter/ receiver means (6) and/or the means of firm attachment to the support means (8) when the probe (1) is manipulated, and in that the means of protection (27) comprise a drop of rigid plastic material (27) which has its maximum thickness facing the rear edge of the piezo-electric disk (6) and which has no surface parallel to the disk (6).

7. Probe according to any one of claims 1 to 6 characterised in that the support means (8) are preferably constituted by a block of uncharged silicon elastomer which is a good ultrasonic conductor, forming a cylindrical or conical section the bases of which form the interior (16) and exterior (17) surfaces of the support means (8), and in that the anterior surface (26) and/or flanks (18) of the support means (8) are inclined and form an angle of less than 90° with the exterior surface (17).

8. Probe according to any one of claims 1 to 7 characterised in that the anterior surface (26) and/or flanks (18) of the support means (8) are at a tangent to the piezo-electric disk.

9. Probe according to any one of claims 1 to 8 characterised in that it comprises means of firm attachment to the organ (15) to be explored, avoiding any untimely subsequent shifting of this probe (1) and in that it comprises two lateral ailerons (25) which facilitate fixing of the probe (1) to the body (15) by means of the firm attachment means on the organ (15), more particularly resorbable wire.

10. Probe according any one of claims 1 to 9 characterised in that the interior surface opposite (16) the support means (8) is inclined at an angle $\alpha$ more particularly of the order of 20° with regard to the exterior surface (17) of these support means (8) in such a way that the ultrasonic waves penetrate into the organ to be explored at an appropriate angle $\theta$, more particularly 60°, with regard to the direction of displacement of the fluid whose speed is to be measured.

11. Probe according to any one of claims 1 to 10 characterised in that the transmitting/receiving surface ( 7) projects downwards with regard to the casing (10) and in that the rear section (30) of this surface ( 7) is inclined to progressively join the casing (10).

## Patentansprüche

1. Sonde mit Ultraschall-Sende-/Empfangsteil zum Einführen in den Körper von Menschen oder Tieren und Sondieren der Innenflächen von Organen (15) durch direkte Berührung mit der Wand eines Organes (15) und Senden/Empfangen von Ultraschallwellen, insbesondere durch Dopplereffekt, bestehend aus Halteteilen (8), piezoelektrischen Sende-/Empfangselementen (6) und einer Umhüllung (10) zur überdeckung der Sende-/Empfangselemente (6) und mindestens den Teil (11) der dem Organ (15) gegenüberliegenden Halteteile (8), wobei die Sende-/Empfangsseite (7) der Sende-/Empfangselemente (6) in direkter Berührung mit einer Fläche (16) der Halteteile (8) ist, welche zwischen die sende-/Empfangselemente (6) und das Organ (15) eingefügt werden, und die Sonde (1) ebenfalls Verbindungselemente (3) zur Außenseite des Körpers besitzt, über die die von der Sonde (1) gesendeten/empfangenen Informationen übertragen werden, wobei die Halteteile (8) eine Außenfläche (17) zum Anlegen an das Gefäß (15) bzw. die Wand eines Organs besitzen, dadurch gekennzeichnet, daß die Halteteile (8) aus einem nicht verstärkten Silikonelastomer und die Umhüllung (10) aus einem verstärkten, Ultraschallwellen gegenüber undurchdringlichen Silikonelastomer bestehen und die Halteteile (8) und die Umhüllung (10) so angeordnet und geformt sind, daß die Sonde ohne komplizierten chirurgischen Eingriff, insbesondere durch einfaches Ziehen an den Verbindungselementen (3) aus dem Körper entfernt werden kann.

2. Sonde nach Patentanspruch 1, dadurch gekennzeichnet, daß die Außenfläche (19) der Umhüllung (10) geglättet und weitgehend tropfenförmig, sich vom Ende (12) des Kabels (4) her zunehmend und gleichmäßig erweiternd ausgebildet ist, an die die Sende-/Empfangselemente (6) angeschlossen sind und daß sie die Sende-/Empfangselemente (6), die elektrischen Anschlüsse (13) sowie die Halteteile (8) ganz oder teilweise überdeckt.

3. Sonde nach einem beliebigen der Patentansprüche 1 oder 2 mit Dichtelementen zur Gewährleistung einer vollkommenen Dichtigkeit der Sonde (1) gegenüber umliegenden Medien, ohne dadurch die mechanischen und akustischen Eigenschaften zu beeinträchtigen, sowie mit elektrischen Isoliermitteln zur vollkommenen elektrischen Isolation der Verbindungselemente (3) und der piezoelektrischen Scheibe (6) nach außen hin, dadurch gekennzeichnet, daß die Mittel zur Gewährleistung der Dichtigkeit und elektrischen Isolierung gemeinsam aus einer dünnen Hülle aus implantierbarem, ultraschalldurchlässigen Material, insbesondere aus nicht verstärktem Silikonelastomer bestehen, welche durch die Halteteile (6), die Umhüllung (10) aus Silikonhaftmittel und das Kabel (4) ganz oder teilweise, insbesondere dessen Ende (12) in der Nähe der Sende-/Empfangselemente (6) durch Anpassung an deren Form überdeckt.

4. Sonde nach einem beliebigen der Patentansprüche 1 bis 3, dadurch gekennzeichnet, daß sie Mittel zur Anpassung der Schallimpedanz zwischen den Sende-/Empfangselementen (6) und den Halteteilen (8) besitzt und daß diese Mittel zur Schallimpedanzanpassung aus einer dünnen Schicht pulverisierten Gemisches auf der Sende-/Empfangsfläche (7) der Sende-/Empfangselemente (6) besteht, durch die insbesondere Störechos bei der Übertragung der Ultraschallwellen zwischen den Halteteilen (8) und den Sende-/Empfangselementen (6) vermieden werden können.

5. Sonde nach Patentanspruch 4, dadurch gekennzeichnet, daß die dünne pulverisierte Schicht aus einer Schicht Farbpulver, wie sie bei der Kaltgalvanisierung verwendet wird, besteht und insbesondere Zink und Epoxyharz enthält.

6. Sonde nach einem beliebigen der Patentansprüche 1 bis 5, dadurch gekennzeichnet, daß sie mit einer Schutzvorrichtung (27) zum Schutz der Sende-/Empfangselemente (6) bzw. der steifen Verbindungselemente (9) zwischen diesen Sende-/Empfangselementen (6) und den Halteteilen (8) ausgestattet ist, um eine Beschädigung der Sende-/Empfangselemente (6) und/oder der steifen Verbindung mit den Halteteilen (8)bei der Benutzung der Sonde (1) zu verhindern und daß die Schutzvorrichtung (27) aus einem Tropfen steifen Kunststoffes (27) besteht, dessen größte Wandstärke sich auf der der Hinterkante der piezoelektrischen Scheibe (6) zugewandten Seite befindet und der keinerlei parallel zu der Scheibe (6) verlaufende Flächen aufweist.

7. Sonde nach einem beliebigen der Patentansprüche 1 bis 6, dadurch gekennzeichnet, daß die Halteteile (8) vorzugsweise aus einem Block nicht verstärkten, Ultraschallwellen gegenüber gut leitfähigen Silokonelastomers bestehen, der die Form eines Zylinders oder Kegelstumpfes hat, dessen Grundfläche die Innenfläche (16) und Außenfläche (17) der Halteteile (8) bildet, sowie dadurch, daß die Außenfläche (26) und/oder die Seitenwände (18) der Halteteile (8) geneigt sind und gegenüber der Außenfläche (17) einen Winkel von 90° bilden.

8. Sonde nach einem beliebigen der Patentansprüche 1 bis 7, dadurch gekennzeichnet, daß die Außenfläche (26) und/oder die Seitenwände (18) der Halteteile (8) tangential zu der piezoelektrischen Scheibe verlaufen.

9. Sonde nach einem beliebigen der Patentansprüche 1 bis 8, dadurch gekennzeichnet, daß sie steife Verbindungsmittel zu dem zu sondierenden Organ (15) besitzt, durch die ein nachträgliches ungewolltes Verlagern der Sonde (1) verhindert wird, sowie dadurch, daß sie zwei seitliche Flügel (25) besitzt, durch die die Befestigung der Sonde (1) an der Wand des Organs (15) durch die steifen Verbindungselemente am Organ (15), insbesondere resorbierbare Fäden, erleichtert wird.

10. Sonde nach einem beliebigen der Patentansprüche 1 bis 9, dadurch gekennzeichnet, daß die gegenüberliegende Innenseite (16) der Halteteile (8) um einen Winkel $\alpha$, insbesondere um 20° gegenüber der Außenseite (17) der Halteteile (8) geneigt ist, sodaß die Ultraschallwellen unter einem geeigneten Winkel $\theta$, insbesondere 60° gegenüber der Flußrichtung des Mediums, dessen Geschwindigkeit gemessen werden soll, in das zu sondierende Organ eindringen.

11. Sonde nach einem beliebigen der Patentansprüche 1 bis 10, dadurch gekennzeichnet, daß die Sende-/Empfangsfläche (7) gegenüber der Umhüllung (10) nach unten vorspringt und der hintere Teil (30) dieser Fläche (7) geneigt ist und so allmählich mit der Umhüllung (10) zusammenläuft.

FIG.1

FIG.2